(19) Europäisches Patentamt
European Patent Office
Office européen des brevets

(11) **EP 3 600 201 B1**

(12) **EUROPEAN PATENT SPECIFICATION**

(45) Date of publication and mention
of the grant of the patent:
**26.05.2021 Bulletin 2021/21**

(21) Application number: **18711178.6**

(22) Date of filing: **20.02.2018**

(51) Int Cl.:
**A61F 13/53** (2006.01)     **C08L 23/08** (2006.01)

(86) International application number:
**PCT/JP2018/005845**

(87) International publication number:
**WO 2018/179995 (04.10.2018 Gazette 2018/40)**

(54) **ABSORBENT BODY AND ABSORBENT ARTICLE COMPRISING THE SAME**

SAUGFÄHIGER KÖRPER UND SAUGFÄHIGER ARTIKEL DAMIT

CORPS ABSORBANT ET ARTICLE ABSORBANT LE COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2017 JP 2017067447**

(43) Date of publication of application:
**05.02.2020 Bulletin 2020/06**

(73) Proprietor: **Livedo Corporation**
**Shikokuchuo-shi, Ehime 799-0122 (JP)**

(72) Inventors:
• **OTA, Yoshihisa**
**Osaka-shi**
**Osaka 541-0048 (JP)**

• **NISHIDA, Motoko**
**Mima-gun**
**Tokushima 779-4104 (JP)**

(74) Representative: **Müller-Boré & Partner**
**Patentanwälte PartG mbB**
**Friedenheimer Brücke 21**
**80639 München (DE)**

(56) References cited:
**EP-A1- 3 147 331      JP-A- H06 245 958**
**US-A1- 2007 135 554**

**Description**

**Technical Field**

The present invention relates to an absorbent body comprising a water absorbent resin powder, and particularly relates to a technique for improving water absorbent resin powder supporting property.

**Background Art**

An absorbent article such as a disposable diaper, sanitary napkin, and incontinence pad comprises an absorbent body. The absorbent body comprises a water absorbent resin powder and the water absorbent resin powder absorbs and retains body fluid excreted from body such as urine and menstrual blood. Among such the absorbent bodies, an absorbent body in which a water absorbent resin powder is fixed on a base material for preventing scattering and deviation of the water absorbent resin powder, is known.

For example, the Patent Literature 1 discloses a super thin absorbent sheet in which a water absorbent resin powder is adhered on one surface of a first nonwoven fabric by a hot melt adhesive in a manner that a water absorbent resin powder existence region and a water absorbent resin powder non-existence region exist (refer to claim 1 of the Patent Literature 1). As disclosed in the Patent Literature 1, as the adhesive for fixing the water absorbent resin powder, a material having rubber elasticity such as elastomer and rubber is often used (refer to lines 13 to 22, page 15 of the Patent Literature 1).

The water absorbent resin takes body fluid inside voids between the polymer chains thereof, and thus the water absorbent resin swells if it absorbs body fluid. In other words, in order to absorb body fluid, it is necessary for the water absorbent resin to swell. However, if the water absorbent resin powder is fixed by an adhesive having rubber elasticity as disclosed in the Patent Literature 1, the swelling of the water absorbent resin powder is hindered by the adhesive. In this case, the original absorption performance of the water absorbent resin powder cannot be fully exerted, and thus the absorption performance of the absorbent body is lowered. Thus, a technology of using a fixing agent that does not hinder the swelling of the water absorbent resin powder has been proposed. For example, the Patent Literature 2 discloses an absorbent body comprising a base material and a water absorbent resin powder fixed on the base material by a fixing agent, wherein the fixing agent has a penetration degree (JIS K 2235) of 10 or less (refer to claim 1, paragraphs 0016, 0017, 0101 of the Patent Literature 2).

**Citation List**

**Patent Literature**

   PTL 1: International Patent Publication No. WO01/089439
   PTL 2: Japanese Patent Publication No. 2015-100610

**Summary of Invention**

**Technical Problem**

The Patent Literature 2 has proposed to use a fixing agent not hindering the swelling of the water absorbent resin powder. However, there is still room for improvement in the water absorbent resin powder supporting property. The present invention has been made in view of the above described circumstances, and an objective of the present invention is to provide an absorbent body comprising a water absorbent resin powder fixed on a base material by a resin and showing excellent water absorbent resin powder supporting property.

**Solution to Problem**

The present invention provides an absorbent body as defined in claim 1.

The present invention further includes a water absorbent resin powder used in the above absorbent body, wherein a water absorbent resin particle constituting the water absorbent resin powder is coated with the fixing agent. In addition, the present invention also includes an absorbent article comprising the above absorbent body.

## Advantageous Effects of the Invention

According to the present invention, an absorbent body comprising a water absorbent resin powder fixed on a base material by a resin and having excellent water absorbent resin powder supporting property, is obtained.

## Brief Description of Drawings

[fig.1]Fig. 1 is a planar view of one example of the absorbent article according to the present invention.
[fig.2]Fig. 2 is a schematic sectional view along line I - I in Fig. 1.
[fig.3]Fig. 3 is a schematic sectional view along line II - II in Fig. 1.

## Description of Embodiments

The present invention provides an absorbent body comprising: a base material and a water absorbent resin powder fixed on the base material by a fixing agent, wherein the fixing agent is a copolymer having an acid value in a range from 20 mgKOH/g to 150 mgKOH/g and including a structural unit (X) derived from at least one monomer selected from the group consisting of an olefin and a vinyl aromatic compound and a structural unit (Y) derived from a monomer having an acidic group.

The fixing agent consisting of the above mentioned copolymer substantially has no rubber elasticity. Such the fixing agent is easily broken when it is stretched. When such the fixing agent is used to fix the water absorbent resin powder, the fixing agent coating the water absorbent resin powder is easily broken by the swelling of the water absorbent resin powder. Thus, the swelling of the water absorbent resin powder is not hindered by the fixing agent, and the absorption performance of the water absorbent resin powder can be fully exerted. In addition, since the fixing agent is a copolymer having a specific acid value and including the structural unit (X) and the structural unit (Y), the fixing agent has high affinity to the base material and high affinity to the water absorbent resin powder as well. Therefore, if the fixing agent is used, an absorbent body showing excellent water absorbent resin powder supporting property, is obtained.

Fixing agent

The fixing agent includes a structural unit (X) derived from at least one monomer (x) selected from the group consisting of an olefin and a vinyl aromatic compound and a structural unit (Y) derived from a monomer (y) having an acidic group. The structural unit (X) has high affinity to the fiber base material. In particular, when using polyethylene, polypropylene or polyethylene telephthalate as the material of the fiber base material, the structural unit (X) has much higher affinity to the fiber base material. The structural unit (Y) has high affinity to the water absorbent resin powder. Thus, if the fixing agent is used, the water absorbent resin powder supporting property on the fiber base material improves. The monomer (x) for forming the structural unit (X) may be used solely, or at least two of them may be used in combination.

As the olefin, an $\alpha$-olefin is preferred. The $\alpha$-olefin is an alkene having a carbon-carbon double bond at $\alpha$ position. The $\alpha$-olefin preferably has 2 to 20 carbon atoms, more preferably 2 to 10 carbon atoms, and even more preferably 2 to 6 carbon atoms. Examples of the $\alpha$-olefin include ethylene, propylene, 1-butene, 1-pentene, 1-hexene, 1-heptene, 1-octene, 1-nonene and 1-decene, and among them, ethylene is particularly preferred.

The vinyl aromatic compound is a compound having an aromatic ring and a vinyl group directly bonding to the aromatic ring. Examples of the vinyl aromatic compound include styrene, $\alpha$-methylstyrene, vinyltoluene, $\alpha$-methyl-4-methylstyrene, dimethylstyrene, ethylstyrene, propylstyrene, isopropylstyrene, butylstyrene, vinylnaphthalene, and 1,1-diphenylethylene. Among them, styrene, $\alpha$-methylstyrene and vinyltoluene are preferred.

As the monomer (x) for forming the structural unit (X), the olefin is preferred. The amount of the structural unit derived from the olefin in the structural unit (X) is preferably 50 mass % or more, more preferably 70 mass % or more, and even more preferably 90 mass % or more. It is also preferred that the structural unit (X) consists of the structural unit derived from the olefin.

The monomer (y) having an acidic group has one carbon-carbon double bond and at least one acidic group in the molecule. Examples of the acidic group include carboxy group (-COOH), sulfonic acid group ($-SO_3H$), phosphoric acid group ($-OPO_3H_2$), phosphonic acid group ($-PO_3H_2$) and phosphinic acid group ($-PO_2H_2$), and among them, the carboxy group is preferred.

As the monomer (y) having an acidic group, an $\alpha,\beta$-unsaturated carboxylic acid having 3 to 10 carbon atoms is preferred. Examples of the monomer (y) having an acidic group include a monomer having one carboxy group such as acrylic acid, methacrylic acid and crotonic acid; a monomer having two carboxy groups such as maleic acid, fumaric acid, chloromaleic acid and itaconic acid; and anhydrides thereof. Among them, at least one member selected from the group consisting of acrylic acid, methacrylic acid, maleic acid, fumaric acid and maleic anhydride is preferred, maleic anhydride is more preferred. The monomer (y) having an acidic group may be used solely, or at least two of them may be used in combination.

The amount of the structural unit (Y) in the copolymer is preferably 0.4 mass % or more, more preferably 0.5 mass % or more, and even more preferably 0.6 mass % or more, and is preferably 5.0 mass % or less, more preferably 4.5 mass % or less, and even more preferably 4.0 mass % or less. If the amount of the structural unit (Y) falls within the above range, the water absorbent resin powder supporting property on the base material is further enhanced.

The copolymer may further include a structural unit other than the structural unit (X) and the structural unit (Y). In this case, the amount of the other structural unit in all the structural units is preferably 30 mass % or less, more preferably 20 mass % or less, even more preferably 10 mass % or less, and most preferably 5 mass % or less. It is also preferred that the copolymer consists of the structural unit (X) and the structural unit (Y). Examples of the monoer for forming the other structural unit include vinylamide and (meth)acrylic amide.

The structure of the copolymer is not particularly limited as long as the copolymer has the structural unit (X) and the structural unit (Y). The copolymer may be any one of a random copolymer, a block copolymer and a graft copolymer. Among them, a graft copolymer including a main chain having the structural unit (X) and a side chain having the structural unit (Y) is preferred.

When the copolymer is the graft copolymer, the amount of the structural unit (X) in all the structural units constituting the main chain is preferably 50 mass % or more, more preferably 80 mass % or more, and even more preferably 90 mass % or more. It is preferred that the main chain does not have the structural unit (Y). It is also preferred that the main chain consists of the structural unit (X). In addition, the amount of the structural unit (Y) in all the structural units constituting the side chain is preferably 50 mass % or more, more preferably 80 mass % or more, and even more preferably 90 mass % or more. It is preferred that the side chain does not have the structural unit (X). It is also preferred that the side chain consists of the structural unit (Y).

When the copolymer is the graft copolymer, it is also preferred that the monomer (y) having an acidic group consists of maleic anhydride. In other words, it is also preferred that the structural unit (Y) consists of the structure derived from maleic anhydride. This is because it is difficult for maleic anhydride to grow into a side chain (graft chain), and thus formation of a ring structure caused by the connection of side chains is suppressed.

The acid value of the copolymer is 50 mgKOH/g or more, and is 130 mgKOH/g or less, and even more preferably 120 mgKOH/g or less. If the acid value of the copolymer falls within the above range, the water absorbent resin powder supporting property on the base material is further enhanced.

The viscosity average molecular weight of the fixing agent is preferably 500 or more, more preferably 600 or more, and even more preferably 700 or more, and is preferably 80000 or less, more preferably 70000 or less, and even more preferably 60000 or less. If the viscosity average molecular weight is 500 or more, fixation of the fixing agent is better, and if the viscosity average molecular weight is 80000 or less, the mechanical strength of the fixing agent is not too high and thus the fixing agent is easily broken by the swelling force of the water absorbent resin powder, as a result of which the hinderance of the fixing agent on the absorptivity of the water absorbent resin powder is further lowered.

The melting temperature of the fixing agent is preferably 50 degrees centigrade or more, more preferably 55 degrees centigrade or more, and even more preferably 60 degrees centigrade or more, and is preferably 160 degrees centigrade or less, more preferably 155 degrees centigrade or less, and even more preferably 150 degrees centigrade or less. If the melting temperature is 50 degrees centigrade or more, the fixing agent hardly melts even if the absorbent body is exposed to a high temperature, and if the melting temperature is 160 degrees centigrade or less, the fixing agent can be fixed at a relative low temperature, and thus deterioration of the base material can be suppressed when the water absorbent resin powder is fixed on the base material. The melting temperature is measured by using a differential scanning calorimeter (DSC).

The fixing agent is obtained by polymerizing the monomer (x) for forming the structural unit (X) and the monomer (y) having an acidic group for forming the structural unit (Y). Examples of the polymerization method include a method of random polymerizing a monomer composition containing the monomer (x) and the monomer (y) having an acidic group; and a method of graft polymerizing the monomer (y) having an acidic group to a polymer of the monomer (x).

In light of the coating property of the fixing agent on the water absorbent resin powder, a production method by which a component that is hardly thermally melted, such as a gel, is not generated in the copolymer, is preferred. Thus, a method of graft polymerizing the monomer (y) having an acidic group to the polymer of the monomer (x) by using an organic peroxide-based radical polymerization initiator in the presence of a radical polymerization chain transfer agent, is particularly preferred.

When carrying out the graft polymerization, a solvent may be used. Examples of the solvent include those capable of dissolving the polymer of the monomer (x) such as normal paraffin or isoparaffin having 7 to 12 carbon atoms, toluene, xylene, diethylbenzene and chlorobenzene. In order to enhance the grafting efficiency, it is preferred that the solvent is not used. When the solvent is not used, the grafting reaction is carried out by heating and melting the polymer of the monomer (x), followed by heating the melted liquid until the melted liquid has a viscosity that can be stirred. It should be noted that when the polymer of the monomer (x) has a number average molecular weight of about fifty thousands or more, the melting liquid has a high viscosity and stirring becomes difficult. In this case, it is preferred to use a kneading extruder or the like.

The radical polymerization chain transfer agent is preferably an alkylmercaptan having 4 to 18 carbon atoms that is a sulfur-containing chain transfer agent with a large chain transfer constant. Specific examples of the radical polymerization chain transfer agent include butylmercaptan, hexylmercaptan, octylmercaptan, nonylmercaptan, decylmercaptan, undecylmercaptan, dodecylmercaptan, tetradecylmercaptan, pentade-cylmercaptan, hexadecylmercaptan, heptadecylmercaptan, and octadecylmercaptan. The amount of the radical polymerization chain transfer agent is from 0.01 part by mass to 15 parts by mass with respect to 100 parts by mass of the monomer (y) having an acidic group.

The organic peroxide-based radical polymerization initiator is not particularly limited, as long as it is a catalyst acting as a polymerization catalyst and showing an effective half-life at a temperature equal to or higher than the temperature at which the polymer of the monomer (x) can be stirred (the temperature at which the viscosity becomes three hundred thousand centipoises or less). As the organic peroxide-based radical polymerization initiator, an organic peroxide-based radical polymerization initiator having a one-hour half-life temperature of 80 degrees centigrade or more is preferable, and an organic peroxide-based radical polymerization initiator having a one-hour half-life temperature of 100 degrees centigrade or more is more preferable. Examples of the organic peroxide-based radical polymerization initiator include a monofunctional polymerization initiator such as t-butylperoxy laurate, t-butylperoxy acetate, t-butyloxy benzoate, dicumyl peroxide and t-butylcumyl peroxide; and a difunctional polymerization initiator such as 1,1-bis(t-butylperoxy)3,3,5-trimethylcyclohexane, 2,5-bis(t-butylperoxy)octane and 2,2-bis(t-butylperoxy)octane, and the monofunctional polymerization initiator is preferred. The amount of the organic peroxide radical polymerization initiator is from 0.1 part by mass to 30 parts by mass with respect to 100 parts by mass of the monomer (y) having an acidic group.

The reaction temperature when carrying out the grafting reaction may be suitably adjusted according to the type of the polymer of the monomer (x), or the used radical polymerization initiator. The reaction temperature is preferably 100 degrees centigrade or more, more preferably 130 degrees centigrade or more, and is preferably 200 degrees centigrade or less, more preferably 180 degrees centigrade or less.

Base material

The absorbent body comprises a base material on which a water absorbent resin powder is fixed. The mass per unit area of the base material is 10 $g/m^2$ or more, preferably 12 $g/m^2$ or more, more preferably 15 $g/m^2$ or more, and is 500 $g/m^2$ or less, preferably 400 $g/m^2$ or less, more preferably 300 $g/m^2$ or less. If the mass per unit area is higher than 500 $g/m^2$, the texture may deteriorate, and if the mass per unit area is lower than 10 $g/m^2$, falling off of the water absorbent resin powder from the nonwoven fabric may occur.

As the base material, a fiber base material composed of a fiber is preferred, and a nonwoven fabric is more preferred. The nonwoven fabric is not particularly limited, any one of a liquid impermeable nonwoven fabric and a liquid impermeable nonwoven fabric may be used. Examples of the nonwoven fabric include point-bond nonwoven fabric, air-through nonwoven fabric, air-laid nonwoven fabric, spun lace nonwoven fabric and spunbond nonwoven fabric, and the air-through nonwoven fabric and the air-laid nonwoven fabric are preferred.

The fineness of the fiber constituting the nonwoven fabric is preferably 1.0 dtex or more, more preferably 1.2 dtex or more, and even more preferably 2.0 dtex or more, and is preferably 5.0 dtex or less, more preferably 4.0 dtex or less, and even more preferably 3.0 dtex or less. If the fineness is 1.0 dtex or more, the nonwoven fabric has suitable voids and thus the liquid permeation speed thereof is better, and if the fineness is 5.0 dtex or less, the voids of the nonwoven fabric are not too large and thus falling off of the water absorbent resin powder is suppressed.

Examples of the fiber constituting the nonwoven fabric include a hydrophobic fiber. Examples of the material of the hydrophobic fiber include polyolefin such as polypropylene and polyethylene; polyester such as polyethylene telephthalate; polyamide; and polyurethane. Among them, at least one member selected from the group consisting of polyethylene, polypropylene and polyethylene telephthalate is preferred. The amount of the hydrophobic fiber in all the fibers constituting the nonwoven fabric is preferably 50 mass% or more, more preferably 70 mass% or more, and even more preferably 90 mass% or more. It is also preferred that the nonwoven fabric consists of the hydrophobic fiber.

The fiber constituting the nonwoven fabric is preferably a crimped fiber, more preferably a spontaneously crimped fiber. If the crimped fiber is used, the water absorbent resin powder is easily entangled with the fiber and thus the fixation of the water absorbent resin powder further enhanced. Examples of the spontaneously crimped fiber include an eccentric core-sheath type spontaneously crimped fiber including a core composed of a first component and a sheath composed of a second component; and a parallel type spontaneously crimped fiber including a first component and a second component. Examples of the combination of the first component and the second component include the combination of polyethylene/ polyethylene telephthalate, the combination of polyamide/polyurethane, and the combination of polyethylene/polypropylene. The first component and the second component in these spontaneously crimped fibers show different thermal contraction from each other, and thus if a heating treatment is conducted, these spontaneously crimped fibers crimp by the thermal contraction difference. For this reason, the spontaneously crimped fiber crimps by the heating treatment conducted when fixing the water absorbent resin powder, and the water absorbent resin powder is hence entrapped inside the nonwoven fabric, thereby further lowering the falling off of the water absorbent resin powder.

Water absorbent resin powder

The water absorbent resin powder is not particularly limited. As the water absorbent resin powder, a crosslinked polymer (A) mainly composed of acrylic acid and having carboxyl groups thereof being at least partially neutralized is preferably. The amount of the acrylic acid component constituting the crosslinked polymer (A) is preferably 90 mass % or more, more preferably 95 mass % or more, and is preferably 99 mass % or less, more preferably 97 mass % or less. If the amount of the acrylic acid component falls within the above range, the obtained water absorbent resin powder can easily exhibit desired absorption performance.

Examples of the cation for neutralizing at least a part of the carboxyl groups of the crosslinked polymer (A) include, but is not particularly limited to, alkali metal ions such as lithium, sodium and potassium, and alkaline earth metal ions such as magnesium and calcium. The neutralization degree of the carboxyl groups of the crosslinked polymer (A) is preferably 60 mole% or more, more preferably 65 mole% or more. This is because if the neutralization degree is too low, the absorption performance of the obtained water absorbent resin powder may deteriorate. In addition, the upper limit of the neutralization degree is not particularly limited, and all the carboxyl groups may be neutralized. It should be noted that the neutralization degree is calculated by the following formula.

$$\text{Neutralization degree (mole\%)} = 100 \times [\text{Number of moles of the neutralized carboxyl groups in the crosslinked polymer}] / [\text{Total number of moles of the carboxyl groups in the crosslinked polymer (including neutralized and unneutralized carboxyl groups)}]$$

The crosslinked polymer (A) is preferably a polymer obtained by polymerizing an unsaturated monomer composition containing: a water-soluble ethylenically unsaturated monomer (a1) and/or a hydrolyzable monomer (a2) producing the water-soluble ethylenically unsaturated monomer (a1) by hydrolysis; and an internal crosslinking agent (b).

As the water-soluble ethylenically unsaturated monomer (a1), for example, a monomer having at least one water-soluble substituent and an ethylenically unsaturated group, or the like may be used. The water-soluble monomer means a monomer having a property of being dissolved in an amount of at least 100 g in 100 g of water at 25 degrees centigrade. In addition, the hydrolyzable monomer (a2) is hydrolyzed with water at 50 degrees centigrade, by the action of a catalyst (an acid or a base) where necessary, to produce the water-soluble ethylenically unsaturated monomer (a1). The hydrolysis of the hydrolyzable monomer (a2) may be conducted during or after the polymerization of the crosslinked polymer or both during and after the polymerization of the crosslinked polymer. However, the hydrolysis of the hydrolyzable monomer (a2) is preferably conducted after the polymerization of the crosslinked polymer in light of the molecular weight of the obtained water absorbent resin powder and the like.

Examples of the water-soluble substituent include a carboxy group, a sulfo group, a sulfoxy group, a phosphono group, a hydroxy group, a carbamoyl group, an amino group, and salts thereof and an ammonium salt. The salt of the carboxy group (carboxylate), the salt of the sulfo group (sulfonate), and the ammonium salt are preferred. In addition, examples of the salt include salts of alkali metal such as lithium, sodium and potassium, and salts of alkaline earth metal such as magnesium and calcium. The ammonium salt may be any one of salts of primary to tertiary amines and salts of quaternary ammonium. Among these salts, in light of absorption properties, the alkali metal salts and the ammonium salts are preferred, the alkali metal salts are more preferred, and the sodium salts are even more preferred.

As the water-soluble ethylenically unsaturated monomer having a carboxy group and/or a salt thereof, an unsaturated carboxylic acid having 3 to 30 carbon atoms and/ or a salt thereof is preferred. Specific examples of the water-soluble ethylenically unsaturated monomer having a carboxy group and/or a salt thereof include unsaturated monocarboxylic acids and/or salts thereof such as (meth)acrylic acid and (meth)acrylic acid salt; unsaturated dicarboxylic acids and/or salts thereof such as maleic acid and maleic acid salt; and monoalkyl (1 to 8 carbon atoms) esters of unsaturated dicarboxylic acids and/or salts thereof such as maleic acid monobutyl ester. Examples of the water-soluble ethylenically unsaturated monomer having a sulfo group and/or a salt thereof include vinyl sulfonic acid and styrene sulfonic acid.

The hydrolyzable monomer (a2) is preferably, but is not particularly limited to, an ethylenically unsaturated monomer having at least one hydrolyzable substituent that becomes a water-soluble substituent by hydrolysis. Examples of the hydrolyzable substituent include a group containing an acid anhydride, a group containing an ester bond, and a cyano group. Examples of the ethylenically unsaturated monomer having a group containing an acid anhydride include an unsaturated dicarboxylic anhydride having 4 to 20 carbon atoms. Examples of the ethylenically unsaturated monomer having a group containing an ester bond include lower alkyl esters of ethylenically unsaturated carboxylic acids and esters of ethylenically unsaturated alcohols. Examples of the ethylenically unsaturated monomer having a cyano group include vinyl group-containing nitrile compounds such as (meth)acrylonitrile.

Each of the water-soluble ethylenically unsaturated monomer (a1) and the hydrolyzable monomer (a2) may be used solely, or a mixture of at least two of them may be used. The same applies to the case where the water-soluble ethylenically unsaturated monomer (a1) and the hydrolyzable monomer (a2) are used in combination.

As the monomer for forming the crosslinked polymer (A), in addition to the water-soluble ethylenically unsaturated monomer (a1) and the hydrolyzable monomer (a2), other vinyl monomer (a3) that is copolymerizable with these monomers may be used. As the other vinyl monomer (a3), hydrophobic vinyl monomers and the like may be used, but it is not limited to them. It should be noted that in light of absorption properties, the amount of the other vinyl monomer (a3) is preferably 0 mole %.

Examples of the internal crosslinking agent (b) include an internal crosslinking agent (b1) having two or more ethylenically unsaturated groups, an internal crosslinking agent (b2) having: at least one functional group that can react with the water-soluble substituent of the water-soluble ethylenically unsaturated monomer (a1) and/or the water-soluble substituent produced by hydrolysis of the hydrolyzable monomer (a2); and at least one ethylenically unsaturated group, and an internal crosslinking agent (b3) having at least two functional groups that can react with the water-soluble substituent of the water-soluble ethylenically unsaturated monomer (a1) and/or the water-soluble substituent produced by hydrolysis of the hydrolyzable monomer (a2). These internal crosslinking agents may be used solely, or at least two of them may be used in combination.

Examples of the internal crosslinking agent (b1) include bis(meth)acrylamides having 8 to 12 carbon atoms, poly(meth)acrylates of polyols having 2 to 10 carbon atoms, polyallylamines having 2 to 10 carbon atoms, and poly(meth)allyl ethers of polyols having 2 to 10 carbon atoms. Specific examples of them include N,N'-methylene bis(meth)acrylamide, ethylene glycol di(meth)acrylate, poly (polymerization degree: 2 to 5) ethylene glycol di(meth)acrylate, propylene glycol di(meth)acrylate, glycerol (di or tri)acrylate, trimethylol propane triacrylate, diallylamine, triallylamine, triallylcyanurate, triallylisocyanurate, tetraallyloxyethane, pentaerythritol diallyl ether, pentaerythritol triallyl ether, pentaerythritol tetraallyl ether, and diglycerin di(meth)acrylate.

Examples of the internal crosslinking agent (b2) include ethylenically unsaturated compounds having 6 to 8 carbon atoms and an epoxy group, ethylenically unsaturated compounds having 4 to 8 carbon atoms and a hydroxy group, and ethylenically unsaturated compounds having 4 to 8 carbon atoms and an isocyanato group.

Examples of the internal crosslinking agent (b3) include polyhydric alcohols, polyvalent glycidyls, polyvalent amines, polyvalent aziridines, and polyvalent isocyanates.

As the method for polymerizing the crosslinked polymer (A), a conventionally known method may be used, and a solution polymerization method, an emulsion polymerization method, a suspension polymerization method, and a reversed-phase suspension polymerization method may be used. In addition, the polymerization liquid when performing the polymerization may be in a form of a thin film, mist, or the like. As the method for controlling the polymerization, an adiabatic polymerization method, a temperature-controlled polymerization method, an isothermal polymerization method, and the like may be used. When the suspension polymerization method or the reversed-phase suspension polymerization method is employed as the polymerization method, conventionally known dispersants, protective colloids, and the like may be used where necessary. In addition, in the case of the reversed-phase suspension polymerization method, the polymerization may be conducted by using a solvent such as hexane. As the polymerization method, the solution polymerization method is preferred, and an aqueous solution polymerization method is more preferred since an organic solvent or the like is not necessarily used and it is advantageous in terms of production cost.

The water-containing gel {consisting of the crosslinked polymer and water} obtained by the polymerization may be chopped where necessary. The size (largest diameter) of the chopped gel is preferably from 50 micrometers to 10 cm. If the size falls within the above range, dryability during a drying process becomes further favorable. The chopping may be conducted by a conventionally known method, and may be conducted by using a conventional chopping apparatus such as a Bexmill, a rubber chopper, a Pharma Mill, a mincing machine, an impact type mill, and a roll type mill.

When a solvent (an organic solvent, water, etc.) is used for the polymerization, it is preferred to remove the solvent by distillation after the polymerization. When the solvent contains an organic solvent, the amount (mass %) of the organic solvent with respect to the mass (100 mass %) of the crosslinked polymer after the removal by distillation is preferably from 0 mass % to 10 mass %. If the amount of the organic solvent falls within the above range, the absorbing performance (in particular, water retention amount) of the water absorbent resin powder becomes further favorable.

As the method for removing the solvent (including water) by distillation, a method of conducting the distillation (drying) by hot air at a temperature in a range from 80 degrees centigrade to 230 degrees centigrade, a thin film drying method of using a drum dryer or the like heated at a temperature in a range from 100 degrees centigrade to 230 degrees centigrade, a (heating) reduced-pressure drying method, a freeze-drying method, a drying method of employing infrared rays, decantation, filtration, and the like may be used.

The crosslinked polymer (A) may be pulverized after being dried. The pulverizing method is not particularly limited, and, for example, an ordinary pulverizing apparatus such as a hammer type pulverizer, an impact type pulverizer, a roll type pulverizer, and a jet streaming type pulverizer may be used. The particle size of the pulverized crosslinked polymer (A) may be adjusted by sieving or the like where necessary.

The mass average particle size (micrometers) of the crosslinked polymer (A) that is sieved where necessary is preferably 200 micrometers or more, more preferably 250 micrometers or more, and even more preferably 300 micrometers or more, and is preferably 550 micrometers or less, more preferably 500 micrometers or less, and even more preferably 450 micrometers or less. If the mass average particle size (micrometers) of the crosslinked polymer (A) falls within the above range, the absorbing performance becomes further favorable.

It should be noted that the mass average particle size is measured with a ro-tap test sieve shaker and standard sieves (JIS Z8801-1: 2006) according to the method described in Perry's Chemical Engineers Handbook, Sixth Edition (The McGraw-Hill Companies, 1984, Page 21). In other words, as JIS standard sieves, sieves of 1000 micrometers, 850 micrometers, 710 micrometers, 500 micrometers, 425 micrometers, 355 micrometers, 250 micrometers, 150 micrometers, 125 micrometers, 75 micrometers, and 45 micrometers, and a tray are combined in order from above. About 50 g of a measuring particle is placed into the uppermost sieve, and shaken with the ro-tap test sieve shaker for 5 minutes. The masses of the measuring particle on each sieve and the tray are measured, and the mass fraction of the particle on each sieve is obtained with the total mass regarded as 100 mass %. The values are plotted in a log probability paper {the horizontal axis is the opening of the sieve (particle size) and the vertical axis is the mass fraction}, followed by connecting each point to draw a line, and a particle size corresponding to 50 mass % of the mass fraction is obtained and regarded as a mass average particle size.

In addition, the lower the amount of fine particles is, the more favorable the absorbing performance becomes. Thus, the amount of fine particles having a size of 150 micrometers or less in all the particles is preferably 10 mass % or less, more preferably 5 mass % or less, and even more preferably 3 mass % or less. The amount of fine particles may be obtained by using the plot created when the above mass average particle size is obtained. Furthermore, from the viewpoint of the fixation of the water absorbent resin powder on the base material, the amount of fine particles having a size of larger than 850 micrometers in all the particles is preferably 10 mass % or less, more preferably 5 mass % or less, and even more preferably 3 mass % or less.

The crosslinked polymer (A) may be subjected to surface crosslinking where necessary. As a crosslinking agent for conducting the surface crosslinking (a surface crosslinking agent), the same ones as the internal crosslinking agent (b) may be used. In light of the absorption performance or the like of the water absorbent resin powder, the surface crosslinking agent is preferably the crosslinking agent (b3), more preferably a polyvalent glycidyl, even more preferably ethylene glycol diglycidyl ether and glycerin diglycidyl ether, and most preferably ethylene glycol diglycidyl ether.

In the case of conducting the surface crosslinking, the amount (mass %) of the surface crosslinking agent with respect to the total mass (100 mass %) of the water-soluble ethylenically unsaturated monomer (a1) and/or the hydrolyzable monomer (a2), the internal crosslinking agent (b), and the other vinyl monomer (a3) used where necessary is preferably from 0.001 mass % to 7 mass %, more preferably from 0.002 mass % to 5 mass %, and even more preferably 0.003 mass % to 4 mass %. In other words, in this case, the upper limit of the amount (mass %) of the surface crosslinking agent based on the total mass of (a1) and/or (a2), (b), and (a3) is preferably 7 mass %, more preferably 5 mass %, and even more preferably 4 mass %. Similarly, the lower limit of the amount (mass %) of the surface crosslinking agent based on the total mass of (a1) and/or (a2), (b), and (a3) is preferably 0.001 mass %, more preferably 0.002 mass %, and even more preferably 0.003 mass %. If the amount of the surface crosslinking agent falls within the above range, the absorption performance becomes further favorable. The surface crosslinking may be achieved by, for example, a method of spraying an aqueous solution containing the surface crosslinking agent to the water absorbent resin powder or impregnating the water absorbent resin powder with the aqueous solution containing the surface crosslinking agent, followed by a heating treatment (100 to 200 degrees centigrade).

The crosslinked polymer (A) may be one polymer or a mixture of two or more polymers. The crosslinked polymer (A) may be further treated with a surface modifier (B). Examples of the surface modifier (B) include a polyvalent metal compound (B1) (e.g. aluminum sulfate, potassium alum, ammonium alum, sodium alum, (poly) aluminum chloride, and hydrates thereof); a polycation compound (B2) (e.g. polyethyleneimine, polyvinylamine, and polyallylamine); an inorganic fine particle (B3); a surface modifier (B4) containing a hydrocarbon group; a surface modifier (B5) containing a hydrocarbon group having a fluorine atom; and a surface modifier (B6) having a polysiloxane structure.

From the viewpoint of the water absorbent resin powder supporting property on the base material, the surface modifier (B) is preferably the inorganic fine particle (B3). It should be noted that the surface modifier (B) preferably does not contain the surface modifier (B5) containing a hydrocarbon group having a fluorine atom and the surface modifier (B6) having a polysiloxane structure, otherwise the water absorbent resin powder supporting property on the base material may tend to be lowered.

Examples of the inorganic fine particle include oxides such as silicon oxide (silica), aluminum oxide (alumina), iron oxide, titanium oxide, magnesium oxide, and zirconium oxide; carbides such as silicon carbide and aluminum carbide; nitrides such as titanium nitride; and complexes thereof (e.g., zeolite, talc). Among them, oxides are preferred, and silicon oxide is more preferred. The volume average particle size of the inorganic fine particle is preferably from 10 nm to 500 nm, without any limitation. The specific surface area of the inorganic fine particle is preferably from 20 $m^2$/g to 4000 m $^2$/g. If the specific surface area falls within the above range, the absorbing performance becomes further favorable. It should be noted that the specific surface area is measured according to JIS Z8830:2001 (nitrogen, a volume method, a multipoint method).

The method for treating the crosslinked polymer (A) with the surface modifier (B) is not particularly limited, as long as the treatment is conducted such that the surface modifier (B) is present on the surface of the crosslinked polymer (A). However, it is preferred that the surface modifier (B) is not contained inside the crosslinked polymer (A). Thus, it is preferred that the surface modifier (B) is mixed with a dried product of the crosslinked polymer (A), rather than being mixed with the water-containing gel of the crosslinked polymer (A) or the polymerization liquid prior to the polymerization of the crosslinked polymer (A). It should be noted that a uniform mixing is preferred.

The shape of the water absorbent resin powder is not particularly limited, and examples thereof include an indefinite crushed shape, a scale shape, a pearl shape, and a rice grain shape. Among them, the indefinite crushed shape is preferred from the standpoint that the powder in such a shape can be well entangled with the fibrous materials in applications such as a disposable diaper and there is little possibility of the powder falling off from the fibrous materials.

The water absorbent resin powder may contain additives such as an antiseptic, a fungicide, an antibacterial, an antioxidant, a ultraviolet absorber, a coloring agent, a perfuming agent, a deodorizer, an inorganic powder, and an organic fibrous material. Examples of such additives include those exemplified in Japanese Patent Publication No. 2003-225565 and Japanese Patent Publication No. 2006-131767. When these additives are contained, the amount (mass %) of the additives with respect to the crosslinked polymer (A) (100 mass %) is preferably from 0.001 mass % to 10 mass %, more preferably from 0.01 mass % to 5 mass %, even more preferably from 0.05 mass % to 1 mass %, and most preferably from 0.1 mass % to 0.5 mass %.

The absorption ratio of the water absorbent resin powder is preferably 40 g/g or more, more preferably 42 g/g or more, and even more preferably 45 g/g or more, and is preferably 80 g/g or less. The absorption ratio is a measure indicating how much water the water absorbent resin powder can absorb. If the absorption ratio is 40 g/g or more, the desirable absorption capacity can be achieved by a small amount of the water absorbent resin powder, and thus the absorbent body can be effectively designed. If the absorption ratio is 80 g/g or less, the stability of the water absorbent resin powder to urine is kept.

The absorption ratio of the water absorbent resin powder under a load is preferably 6 g/g or more, more preferably 10 g/g or more, and even more preferably 15 g/g or more, and is preferably 35 g/g or less, more preferably 33 g/g or less, and even more preferably 30 g/g or less. The absorption ratio under the load is a measure indicating how much water the water absorbent resin powder can absorb under a load of 2 kPa (20 gf/$cm^2$). If the absorption ratio under the load is 6 g/g or more, the desirable absorption capacity can be achieved by a small amount of the water absorbent resin powder, and thus the absorbent body can be effectively designed. If the absorption ratio under the load is 35 g/g or less, the stability of the water absorbent resin powder to urine is kept.

The absorption speed of the water absorbent resin powder measured by a vortex method is preferably 5 seconds or more, more preferably 10 seconds or more, and even more preferably 15 seconds or more, and is preferably 100 seconds or less, more preferably 80 seconds or less, and even more preferably 70 seconds or less. The absorption speed measured by the vortex method is evaluated by measuring time (seconds) required for absorbing body fluid. Thus, a shorter measuring time (seconds) means a greater speed to absorb body fluid by the water absorbent resin powder. If the absorption speed measured by the vortex method is 5 seconds or more, the stability, especially the stability under a load, of the water absorbent resin powder to urine is better. If the absorption speed measured by the vortex method is 100 seconds or less, body fluid can be sufficiently absorbed even when a large amount of body fluid is excreted one time at a high speed.

The bulk density of the water absorbent resin powder is preferably 0.40 g/ml or more, more preferably 0.42 g/ml or more, and even more preferably 0.45 g/ml or more, and is preferably 0.85 g/ml or less, more preferably 0.83 g/ml or less, and even more preferably 0.80 g/ml or less. The bulk density is an index of the shape of the water absorbent resin powder. If the bulk density falls within the above range, a void is easily formed for a passage of body fluid between the water absorbent resin powders. As a result, the absorption speed and the repeated-absorption speed become favorable.

The absorption ratio, absorption speed and bulk density of the water absorbent resin powder can be adjusted by suitably selecting the composition of the crosslinked polymer (A), the type of the surface modifier, the particle size of the water absorbent resin powder, the drying condition, or the like.

Absorbent body

The absorbent body according to the present invention comprises the base material and the water absorbent resin powder fixed on the base material by the fixing agent. The mass of the water absorbent resin powder per unit area in the absorbent body is preferably 20 g/m$^2$ or more, more preferably 60 g/m$^2$ or more, and even more preferably 100 g/m$^2$ or more, and is preferably 500 g/m$^2$ or less, more preferably 400 g/ m$^2$ or less, and even more preferably 300 g/m$^2$ or less. If the mass per unit area is 20 g/ m$^2$ or more, the absorbent body has further enhanced absorbing capacity, and if the mass per unit area is 500 g/m$^2$ or less, the absorbent body has better texture.

The amount of the fixing agent in the absorbent body is preferably 0.3 part by mass or more, more preferably 0.4 part by mass or more, and even more preferably 0.5 part by mass or more, and is preferably 5.0 parts by mass or less, more preferably 4.5 parts by mass or less, and even more preferably 4.0 parts by mass or less, with respect to 100 parts by mass of the water absorbent resin powder. If the amount of the fixing agent is 0.3 part by mass or more, the fixation of the water absorbent resin powder on the base material further enhanced, and if the amount of the fixing agent is 5.0 parts by mass or less, the absorption performance of the water absorbent resin powder is not hindered.

The absorbent body may further comprise a water absorbent material such as a water absorbent fiber, in addition to the above mentioned water absorbent resin powder. Examples of the water absorbent fiber include pulp fibers, cellulose fibers, rayon, and acetate fibers.

Examples of the construction of the absorbent body include an embodiment composed of one sheet of the base material and the water absorbent resin powder fixed on one surface of the base material; and an embodiment composed of two sheets of the base material and the water absorbent resin powder fixed between two sheets of the base material. The water absorbent resin powder fixed on the base material may be uniformly fixed on the whole surface of the base material, or may be fixed in multiple streaks extending continuously (or intermittently) in one direction. The planar view shape of the absorbent body is not particularly limited, and examples thereof include a rectangular shape, an hourglass shape, a gourd shape, and a battledore shape.

The method for producing the absorbent body is not particularly limited, and examples thereof include a method of applying the fixing agent on the base material and spraying the water absorbent resin powder thereon, followed by carrying out a heating treatment; and a method of coating the water absorbent resin particle with the fixing agent and spraying the water absorbent resin particle coated with the fixing agent on the base material, followed by carrying out a heating treatment. It is preferred that the temperature of the heating treatment is not lower than the melting temperature of the fixing agent and is 160 degrees centigrade or less.

The method of coating the water absorbent resin particle with the fixing agent is not particularly limited. The water absorbent resin powder and the fixing agent may be mixed, heated and kneaded to perform the coating. It is preferred that the heating temperature is not lower than the melting temperature of the fixing agent and is 160 degrees centigrade or less. The mixture obtained by the kneading may be molded into a sheet shape, or pulverized after being cooled. The surface of the water absorbent resin particle coated with the fixing agent is at least partially coated with the fixing agent. In the powder consisting of the water absorbent resin particle coated with the fixing agent, the amount of the fixing agent is preferably 0.3 part by mass or more, more preferably 0.4 part by mass or more, and even more preferably 0.5 part by mass or more, and is preferably 5.0 parts by mass or less, more preferably 4.5 parts by mass or less, and even more preferably 4.0 parts by mass or less, with respect to 100 parts by mass of the water absorbent resin powder.

Absorbent article

Next, specific application examples of the absorbent body according to the present invention will be provided. Examples of the absorbent article in which the absorbent body according to the present invention may be used include absorbent articles used for absorbing body fluid excreted from human body, such as an incontinence pad, a disposable diaper, and a sanitary napkin.

When the absorbent article is an incontinence pad or a sanitary napkin, for example, the absorbent body is disposed between a liquid permeable top sheet and a liquid impermeable back sheet. Examples of the shape of the incontinence pad or the sanitary napkin include a substantially rectangular shape, an hourglass shape, and a gourd shape. In addition, a liquid impermeable side sheet may be formed on both sides of the liquid permeable top sheet in the width direction, where necessary. The side sheet is joined to the upper surface of both sides of the top sheet in the width direction, and the side sheet inward of the joining point in the width direction forms one pair of rise flaps along both side edges of the absorbent body.

When the absorbent article is a disposable diaper, examples of the disposable diaper include a tape-type disposable diaper that has one pair of securing members on left and right sides of a back portion or a front abdominal portion, and that forms, because of the securing members, a pants shape when being worn; and a pants-type disposable diaper having a waist opening and one pair of leg openings formed by joining a front abdominal portion and a back portion together.

When the absorbent article is the disposable diaper, in the disposable diaper, for example, a laminated body including an inner sheet and an outer sheet may form a diaper main body including a front abdominal portion, a back portion, and a crotch portion located between these portions, and the absorbent body may be disposed at the crotch portion. Furthermore, the disposable diaper may include, for example, a laminated body having the absorbent body disposed between a top sheet and a back sheet, and the laminated body may include a front abdominal portion, a back portion, and a crotch portion located between these portions. It should be noted that, with regard to the front abdominal portion, the back portion, and the crotch portion, when the disposable diaper is worn, a portion placed on the abdominal side of the wearer is referred to as a front abdominal portion, a portion placed on the hip side of the wearer is referred to as a back portion, and a portion located between the front abdominal portion and the back portion and placed on the crotch of the wearer is referred to as a crotch portion. The inner sheet is preferably hydrophilic or water-repellent, and the outer sheet is preferably water-repellent.

The absorbent article preferably has rise flaps disposed along both side edges of the absorbent body. The rise flaps, for example, may be disposed on both side edges of the top surface of the absorbent body in the width direction, or may be disposed on both outer sides of the absorbent body in the width direction. By disposing the rise flaps, side leakage of body fluid can be prevented. The rise flaps may be formed by causing inward edges of the side sheets provided on both sides of the top sheet in the width direction, to rise. The rise flaps and the side sheets are preferably water-repellent.

Next, the absorbent article according to the present invention will be described with reference to figures. However, the present invention is not limited to the embodiments shown in these figures.

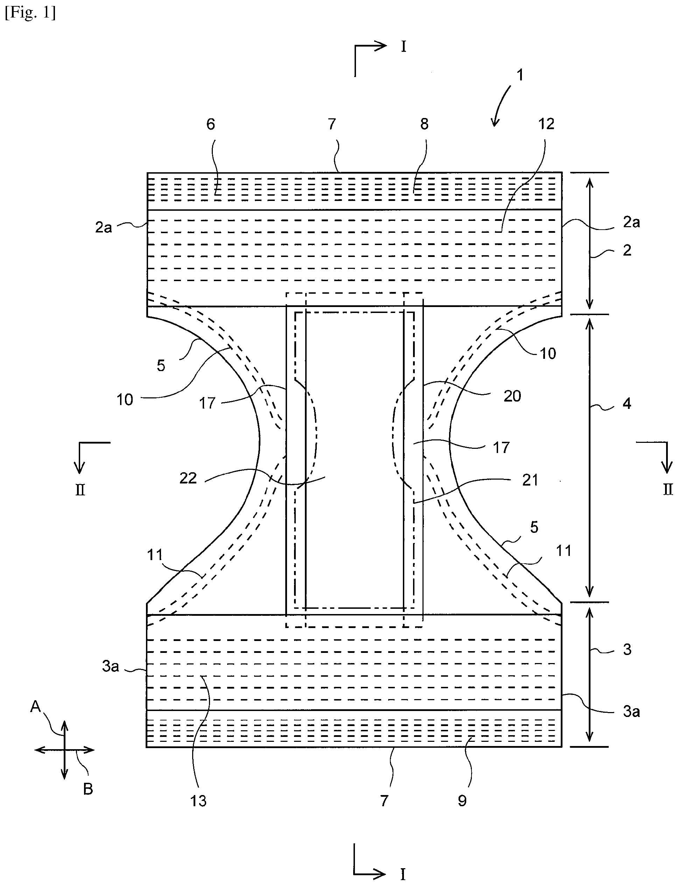

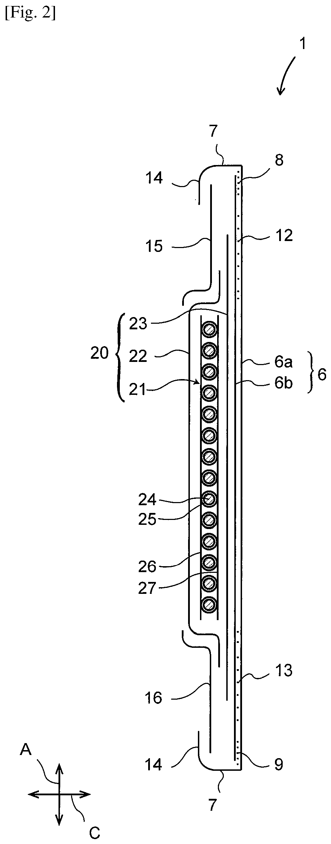

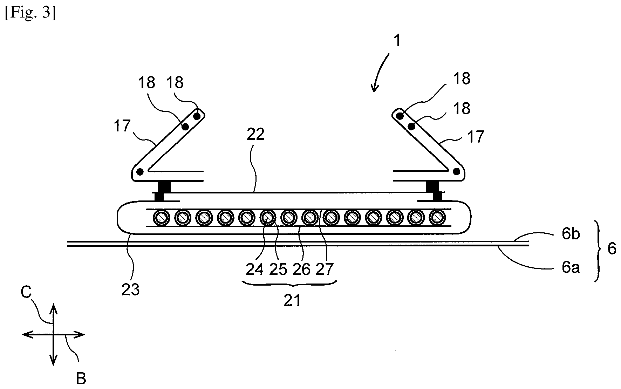

Fig. 1 is a planar view (expansion planar view) of one example of the absorbent article according to the present invention, Fig. 2 is a schematic sectional view along line I - I in Fig. 1, and Fig. 3 is a schematic sectional view along line II - II in Fig. 1. In Fig. 1 to Fig. 3, the arrow A is defined as the length direction of the absorbent article, the arrow B is defined as the width direction of the absorbent article, and with respect to C direction on the paper surface, the upper side is the skin surface side and the lower side is the external surface side.

Fig. 1 shows one example of a pants-type disposable diaper (absorbent article) according to the present invention (expansion planar view). A pants-type disposable diaper 1 comprises a front abdominal portion 2 and a back portion 3 in a length direction A, and a crotch portion 4 disposed between the front abdominal portion 2 and the back portion 3. The front abdominal portion 2 makes contact with the abdominal side of the wearer, and the back portion 3 makes contact with the hip side of the wearer. Notches 5 are provided on the crotch portion 4 along the circumference of the legs of the wearer. In the pants-type disposable diaper 1 shown in Fig. 1, side edges 2a of the front abdominal portion 2 are joined to side edges 3a of the back portion 3 to form the pants-type disposable diaper 1 having a waist opening and one pair of leg openings.

In the pants-type disposable diaper 1, an absorbent main body 20 is attached on the skin surface side of an exterior sheet material 6. The absorbent main body 20 extends in the length direction A from the center of the crotch portion 4.

In the pants-type disposable diaper 1, a front-side waist elastic member 8 and a back-side waist elastic member 9 are attached in a stretched state in the width direction B along end edges 7 of the exterior sheet material 6. In addition, front-side leg elastic members 10 and back-side leg elastic members 11 are attached along the notches 5 in a stretched state. On the front abdominal portion 2 and the back portion 3, a front-side torso circumference elastic member 12 and a back-side torso circumference elastic member 13 are respectively attached in the width direction B in a stretched state between the waist elastic member and the leg elastic member. Through contraction of each of the elastic members, the pants-type disposable diaper 1 fits onto the wearer.

Fig. 2 is a schematic sectional view along line I - I of the pants-type disposable diaper shown in Fig. 1. The construction of the pants-type disposable diaper 1 will be described with reference to Fig. 2. The exterior sheet material 6 includes an outer sheet 6a and an inner sheet 6b, and, between the sheets, the waist elastic members 8 and 9, the leg elastic members 10 and 11, the torso circumference elastic members 12 and 13 are attached in a stretched state. The outer sheet 6a is longer than the inner sheet 6b in the length direction, and has formed thereon folded portions 14 that are folded toward inner surface sides (skin surface side) at the end edges 7.

The absorbent main body 20 is attached on the skin surface side of the exterior sheet material 6. The absorbent main body 20 comprises an absorbent body 21, a top sheet 22 made of a nonwoven fabric material and disposed on the skin surface side of the absorbent body 21, and a liquid impermeable back sheet 23 provided on the external surface side of the absorbent body 21. The absorbent body 21 comprises a water absorbent resin powder 24, a fixing agent 25 coating the water absorbent resin powder 24, and base materials 26, 27 on which the water absorbent resin powder is fixed. In the pants-type disposable diaper 1, a front-side end holding sheet 15 and a back-side end holding sheet 16 are provided at the front abdominal portion 2 and the back portion 3 on the inner surface of the inner sheet 6b so as to cover end portions of the absorbent main body 20 in the length direction.

Fig. 3 is a schematic sectional view along line II - II of the pants-type disposable diaper shown in Fig. 1. As shown in Fig. 3, side sheets 17 made of a nonwoven fabric material are joined on both side-edge portions in the width direction of the top sheet 22 made of a nonwoven fabric material. The side sheets 17 form rise flaps that rise toward the skin of the wearer through the contractive force of an elastic members 18 attached in the length direction in a stretched state, and act as a barrier for preventing side leakage of urine, etc.

Specific examples of the absorbent article according to the present invention include absorbent articles used for absorbing body fluid excreted from human body, such as a disposable diaper, a sanitary napkin, and an incontinence pad.

**Examples**

Next, the present invention will be described in detail by means of examples. However, the present invention is not limited to the examples below, and changes and embodiments that do not depart from the gist of the present invention are all included in the scope of the present invention.

Acid value of fixing agent

The acid value of the fixing agent was measured by a method based on JIS K0070 (1992). Specifically, the fixing agent was dissolved in toluene, phenolphthalein was added into the obtained solution, and 0.1 mol/L of potassium hydroxide ethanol solution was used to conduct neutralization titration. The acid value (A) was calculated by the following formula.

$$A = 5.611 \times B \times f / S$$

A: acid value (mgKOH/g)
B: amount of potassium hydroxide ethanol solution used in the titration (ml)
f: factor of potassium hydroxide ethanol solution
S: mass of sample (g)

Absorption ratio

The absorption ratio was measured according to JIS K 7223 (1996). A nylon mesh having openings of 63 micrometers (JIS Z8801-1: 2000) was cut into a rectangle having a width of 10 cm and a length of 40 cm and folded in half at the center in its length direction, and both ends thereof were heat-sealed to produce a nylon bag having a width of 10 cm (inside dimension: 9 cm) and a length of 20 cm. A measuring sample with a mass of 1.00 g was precisely weighted and placed into the produced nylon bag such that the sample was uniform at the bottom of the nylon bag. The nylon bag having the measuring sample contained therein was immersed into physiological saline. The nylon bag was taken out from the physiological saline 60 minutes later after the start of the immersion, and hung vertically for 1 hour to drain, and then the mass F1 (g) of the sample was measured. In addition, the same operation was conducted without using any sample, and the mass F0 (g) at that time was measured. The target absorption ratio was calculated according to the following formula based on the masses F1, F0 and the mass of the sample.

$$\text{Absorption ratio (g/g)} = (F1 - F0) / \text{mass of sample}$$

Absorption ratio under a load (absorption amount under a load of 2 kPa)

The absorption ratio under a load was measured as follows in an environment of 25 degrees centigrade plus or minus 2 degrees centigrade and a relative humidity of 50 % plus or minus 5 %. Specifically, 0.10 g of a sample was weighted in a cylindrical plastic tube (inner diameter: 30 mm, height: 60 mm) with a nylon mesh having openings of 63 micrometers (JIS Z8801-1: 2000) attached on the bottom thereof. The cylindrical plastic tube was allowed to become vertical such that the sample therein almost had a uniform thickness on the nylon mesh, and a balance weight having an outer diameter of 29.5 mm and a thickness of 22 mm was inserted into the cylindrical plastic tube to apply a load of 2 kPa to the sample. It should be noted that the masses of the cylindrical plastic tube and the balance weight had been measured in advance. Subsequently, the cylindrical plastic tube having the sample and the balance weight placed therein was vertically immersed in a petri dish (diameter: 120 mm) having 60 ml of physiological saline placed therein, in a manner that the nylon mesh side thereof was the lower side. At this time, the cylindrical plastic tube was immersed in a depth that the cylindrical plastic tube nearly came into contact with the bottom of the petri dish. Sixty minutes later, the cylindrical plastic tube having the sample and the balance weight placed therein was taken out from the water and the mass thereof was weighted, and from this mass, the masses of the cylindrical plastic tube and the balance weight measured in advance were subtracted to calculate the mass of the physiological saline absorbed by the sample. A value obtained by multiplying the mass of the absorbed physiological saline by 10 was adopted as the absorption amount under a load (g/g).

Absorption speed measured by a vortex method

50 ml of physiological saline (0.9 mass % sodium chloride solution) and a magnetic stirrer tip (diameter at the center portion: 8 mm, diameter at both end portions: 7 mm, length: 30 mm, surface was coated with a fluororesin) were added into a 100 ml glass beaker, and the beaker was placed on a magnetic stirrer ("HPS-100" available from AS ONE Corporation). The rotational speed of the magnetic stirrer was adjusted to 600 rpm plus or minus 60 rpm to stir the physiological saline. 2.0 g of a sample was added into the solution at the center of the vortex of the physiological saline being stirred, and the absorption speed (seconds) of the water absorbent resin powder was measured according to JIS K 7224 (1996). Specifically, a stopwatch was started at the time when the addition of the water absorbent resin powder, which was the sample, to the beaker was completed, and stopped at the time when the stirrer tip was covered with the test solution (the time when the vortex disappeared and the surface of the solution became flat), and the time (seconds) was recorded as the absorption speed. The measurement was conducted five times (n = 5), the highest and lowest values were removed, and the average of the remaining three values was adopted as the measured value. It should be noted that these measurements were conducted at 23 degrees centigrade plus or minus 2 degrees centigrade and a relative humidity of 50 plus or minus 5%, and the sample was stored in the same environment for at least 24 hours prior to the measurements and then were subjected to the measurements.

Bulk density

The bulk density was measured according to JIS K 6219-2 (2005). A water absorbent resin powder that was a sample was poured into a center portion of a cylindrical container whose mass and capacity were known (a stainless steel container having a diameter of 100 mm and a capacity of 1000 ml), from a height of 50 mm or less from the upper end of the container. At this time, a sufficient amount of the sample was poured into the cylindrical container such that the poured sample formed a triangular pyramid above the upper end of the cylindrical container. Then, the excessive sample above the upper end of the cylindrical container was swept down using a spatula, and the mass of the container in this state was measured. The mass of the container itself was subtracted from the measured value to obtain the mass of the sample, and the mass of the sample was divided by the capacity of the container to calculate the target bulk density. The measurement was conducted five times (n = 5), the highest and lowest values were removed, and the average of the remaining three values was adopted as the measured value. It should be noted that these measurements were conducted at 23 degrees centigrade plus or minus 2 degrees centigrade and a relative humidity of 50 plus or minus 5%, and the sample was stored in the same environment for at least 24 hours prior to the measurements and then were subjected to the measurements.

Synthesis of fixing agent

Fixing agent No. 1 1000.0 g of an ethylene polymer ("RL410P" available from Honeywell Japan Co. Ltd.), 30.0 g of maleic anhydride and 1.5 g of dodecylmercaptan were charged into a reaction vessel, and allowed to be dissolved by heating at 170 degrees centigrade under ventilation of nitrogen gas. The obtained solution was stirred for 1 hour, 6.0 g of dicumyl peroxide was added therein, and the reaction was conducted for 20 minutes to obtain a graft copolymer having an acidic group (the polymer 1). The unreacted maleic anhydride was removed from the polymer 1 by using a heat acetone, and the fixing agent No. 1 was obtained. The fixing agent No. 1 is a graft copolymer including a main chain having the structural unit (X) and a side chain having the structural unit (Y). The amount of the structural unit (X) in all the structural units constituting the main chain is at least 90 mass %, and the side chain consists of the structural unit (Y) (consists of the structure derived from maleic anhydride). The fixing agent No. 1 has an acid value of 20.2 mgKOH/g and a melting temperature of 73 degrees centigrade.

Fixing agents No. 2 to 6

The fixing agents No. 2 to 6 were produced by the same method as the method for producing the fixing agent No. 1 except that the amounts of maleic anhydride, dodecylmercaptan and dicumyl peroxide were changed according to Table 1. The acid value and melting temperature of each fixing agent are described in Table 2.

[Table 1]

| Fixing agent No. | Charging amount (g) | | | | A cid value (mgKOH/g) |
|---|---|---|---|---|---|
| | Ethylene polymer | Maleic anhydride | Dodecylmercaptan | Dicumyl peroxide | |
| 1 | 1000.0 | 30.0 | 1.5 | 6.0 | 20.2 |
| 2 | 1000.0 | 78.0 | 3.9 | 15.6 | 51.3 |
| 3 | 1000.0 | 183.0 | 9.15 | 36.6 | 121.3 |
| 4 | 1000.0 | 230.0 | 11.5 | 46.0 | 148.2 |
| 5 | 1000.0 | 27.0 | 1.35 | 5.4 | 18.5 |
| 6 | 1000.0 | 250.0 | 12.5 | 50.0 | 154.3 |

Fixing agent No. 7

1000.0 g of styrene and 100.0 g of maleic anhydride were charged into a pressure resistant reaction vessel (autoclave), and heated and mixed at 130 degrees centigrade under ventilation of nitrogen gas. Under stirring, a mixture containing 15 g of t-butylperoxy benzoate and 2.5 g of dodecylmercaptan was dropwise added therein by taking 30 hours, and the reaction was conducted at 135 degrees centigrade for 3 hours to obtain a random copolymer having an acidic group (the polymer 7). The unreacted maleic anhydride and styrene were removed from the polymer 7 by using a heat acetone, and the fixing agent No. 7 was obtained. The acid value and melting temperature of the fixing agent No. 7 are described in Table 2.

Fixing agents No. 8 to 13

The following fixing agents were prepared as the fixing agents No. 8 to 13.
Fixing agent No. 8: ethylene-acrylic acid random copolymer ("A-C5120" available from Honeywell Japan Co. Ltd.)
Fixing agent No. 9: ethylene-acrylic acid random copolymer ("A-C580" available from Honeywell Japan Co. Ltd.)
Fixing agent No. 10: ethylene-acrylic acid random copolymer ("A-C540A" available from Honeywell Japan Co. Ltd.)
Fixing agent No. 11: low-molecular-weight polyolefin wax (HI-WAX 4202E (acid value: 17.1 mgKOH/g, melting temperature: 100 degrees centigrade) available from Mitsui Chemicals, Inc.)
Fixing agent No. 12: low-molecular-weight polyolefin wax (EXCEREX (Registered trademark) 15341PA (acid value: 14.0 mgKOH/g, melting temperature: 89 degrees centigrade) available from Mitsui Chemicals, Inc.)
Fixing agent No. 13: synthetic rubber-based hot melt adhesive having an aromatic group in the molecule (MORESCO-MELT TN-260Z available from MORESCO Corporation)

Synthesis of water absorbent resin powder

155 parts by mass of acrylic acid, 0.6225 parts by mass of pentaerythritol triallyl ether and 340.27 parts by mass of deionized water were kept at 1 degree centigrade while being stirred and mixed. After nitrogen was introduced into the mixture to reduce the amount of the dissolved oxygen to 0.1 ppm or less, 0.31 part by mass of a 1 % hydrogen peroxide aqueous solution, 1.1625 parts by mass of a 1 % ascorbic acid aqueous solution and 2.325 parts by mass of a 0.5 % 2,2'-azobis[2-methyl-N-(2-hydroxyethyl)-propionamide] aqueous solution were added therein and mixed to initiate polymerization. After the temperature of the mixture reached 85 degrees centigrade, the polymerization was conducted at 85 degrees centigrade plus or minus 2 degrees centigrade for about 10 hours to obtain a water-containing gel.
Next, while 502.27 parts by mass of the water-containing gel was chopped with a mincing machine, 128.42 parts by mass of a 48.5 % sodium hydroxide aqueous solution was added and mixed, and further 3 parts by mass of a 1 % ethylene glycol diglycidyl ether aqueous solution was added and mixed, to obtain a chopped gel. Further, the chopped gel was dried with an air-flow band dryer to obtain a dried product. The dried product was pulverized with a juicer-mixer, and then the particle size thereof was adjusted to be from 150 micrometers to 710 micrometers by using sieves having openings of 150 micrometers and 710 micrometers, to obtain a dried product particle. While 100 parts by mass of the dried product particle was stirred at a high speed, 5 parts by mass of a 2 % water/methanol mixed solution (mass ratio of water/methanol = 70/30) of ethylene glycol diglycidyl ether was added by spraying and mixed, and the mixture was kept still at 150 degrees centigrade for 30 minutes to conduct surface crosslinking, thereby obtaining the crosslinked polymer (A).

Into 100 parts by mass of the crosslinked polymer (A), 0.5 part by mass of silica (Aerosil 380 available from Toshin Chemicals Co.,Ltd.) was added as the surface modifier (B), followed by stirring at 85 degrees centigrade for 60 minutes. The mass average particle size of the obtained resin powder was adjusted to 400 micrometers to obtain a water absorbent resin powder. The obtained water absorbent resin powder has a bulk density of 0.61 g/ml, absorption ratio of 60 g/g, absorption ratio under a load of 2 kPa of 25 g/g, and absorption speed measured by a vortex method of 50 seconds, in which the proportion of particles having a particle size of 150 micrometers or less is 1.2 mass %, and the proportion of particles having a particle size of larger than 850 micrometers is 0 mass %.

Preparation of absorbent body

Absorbent bodies No. 1 to 12

20 g of the water absorbent resin powder and 0.5 g of the fixing agent were charged into a beaker, and kneaded while being heated at 130 degrees centigrade. Next, the mixture was cooled to 80 degrees centigrade to obtain a water absorbent resin coated with the fixing agent. 2 g of the water absorbent resin coated with the fixing agent was sprayed on a base material (130 mm × 130 mm). As the base material, an air-through nonwoven fabric (mass per unit area: 18 g/m$^2$) was used. The fiber constituting the nonwoven fabric was a core-sheath type fiber (material of the core: polypropylene, material of the sheath: polyethylene) and had a fineness of 2.2 dtex. The water absorbent resin was uniformly sprayed on the nonwoven fabric in an area of 100 mm × 100 mm. After completing the spraying of the water absorbent resin, a heating treatment was conducted by placing the obtained product in an oven of 140 degrees centigrade, to obtain an absorbent body. The fixation of the water absorbent resin powder in each absorbent body was evaluated and shown in Table 2.

Absorbent body No. 13

20 g of the water absorbent resin powder and 0.5 g of the fixing agent were charged into a beaker, and kneaded while being heated at 140 degrees centigrade. Next, the mixture was cooled to 80 degrees centigrade to obtain a water absorbent resin coated with the fixing agent. 2 g of the water absorbent resin coated with the fixing agent was sprayed on a base material (130 mm × 130 mm). As the base material, an air-through nonwoven fabric (mass per unit area: 18 g/m$^2$) was used. The fiber constituting the nonwoven fabric was a core-sheath type fiber (material of the core: polypropylene, material of the sheath: polyethylene) and had a fineness of 2.2 dtex. The water absorbent resin was uniformly sprayed on the nonwoven fabric in an area of 100 mm × 100 mm. After completing the spraying of the water absorbent resin, a heating treatment was conducted by placing the obtained product in an oven of 140 degrees centigrade, to obtain an absorbent body. The fixation of the water absorbent resin powder in each absorbent body was evaluated and shown in Table 2.

Absorbent body No. 14

20 g of the water absorbent resin powder and 2.0 g of the fixing agent were charged into a beaker, and kneaded while being heated at 140 degrees centigrade. Next, the mixture was cooled to 80 degrees centigrade to obtain a water absorbent resin coated with the fixing agent. 2 g of the water absorbent resin coated with the fixing agent was sprayed on a base material (130 mm × 130 mm). As the base material, an air-through nonwoven fabric (mass per unit area: 18 g/m$^2$) was used. The fiber constituting the nonwoven fabric was a core-sheath type fiber (material of the core: polypropylene, material of the sheath: polyethylene) and had a fineness of 2.2 dtex. The water absorbent resin was uniformly sprayed on the nonwoven fabric in an area of 100 mm × 100 mm. After completing the spraying of the water absorbent resin, a heating treatment was conducted by placing the obtained product in an oven of 140 degrees centigrade, to obtain an absorbent body. The fixation of the water absorbent resin powder in each absorbent body was evaluated and shown in Table 2.

Fixation evaluation

Fixing ratio 1

The absorbent body was placed on a sieve (openings: 4 mm) in a manner that the surface thereof on which the water absorbent resin had been sprayed faced down, and sieved 20 times, and the mass of the water absorbent resin falling off from the absorbent body (also including the mass of the fixing agent falling off from the absorbent body) was measured. Next, the fixing ratio was calculated according to the following formula based on the mass of the water absorbent resin coated with the fixing agent, which had been sprayed on the base material, and the mass of the water absorbent resin falling off from the absorbent body.

$$\text{Fixing ratio (mass \%)} = 100 \times \{(\text{mass of the sprayed water absorbent resin coated}$$
$$\text{with the fixing agent}) - (\text{mass of the water absorbent resin falling off from the}$$
$$\text{absorbent body})\} / (\text{mass of the sprayed water absorbent resin coated with the fixing}$$
$$\text{agent})$$

Fixing ratio 2

The fixing ratio 2 was measured by the same method as the method for measuring the fixing ratio 1 except that the sieving time was changed to 40 times.

[Table 2]

| Absorbent body No. | Fixing agent | | | | Evaluation | | |
| | No. | Acid value (mgKOH/g) | Melting temperature (degrees centigrade) | Amount with respect to 100 parts by mass of water absorbent resin (parts by mass) | Fixing ratio 1 | Fixing ratio 2 | Grading |
|---|---|---|---|---|---|---|---|
| 1 | 1 | 20.2 | 73 | 2.5 | 97 | 82 | Good |
| 2 | 2 | 51.3 | 73 | 2.5 | 95 | 92 | Excellent |
| 3 | 3 | 121.3 | 72 | 2.5 | 96 | 94 | Excellent |
| 4 | 4 | 148.2 | 72 | 2.5 | 97 | 82 | Good |
| 5 | 7 | 95.0 | 94 | 2.5 | 94 | 90 | Excellent |
| 6 | 8 | 120.2 | 98 | 2.5 | 93 | 91 | Excellent |
| 7 | 9 | 75.0 | 95 | 2.5 | 95 | 91 | Excellent |
| 8 | 10 | 40.4 | 105 | 2.5 | 91 | 84 | Good |
| 9 | 5 | 18.5 | 73 | 2.5 | 97 | 60 | Fair |
| 10 | 6 | 154.3 | 72 | 2.5 | 95 | 59 | Fair |
| 11 | 11 | 17.1 | 100 | 2.5 | 97 | 65 | Fair |
| 12 | 12 | 14.0 | 89 | 2.5 | 97 | 61 | Fair |
| 13 | 13 | - | 82 | 2.5 | 25 | 21 | Poor |
| 14 | 13 | - | 82 | 10.0 | 41 | 38 | Poor |

The absorbent bodies No. 1 to 8 are the cases where a copolymer having an acid value of from 20 mgKOH/g to 150 mgKOH/g and including the structural unit (X) and the structural unit (Y), is used as the fixing agent. It can be seen that these absorbent bodies have a fixing ratio 2 of at least 82 in the fixation evaluation, and thus have excellent fixation.

The absorbent bodies No. 9 to 12 are the cases where a copolymer having an acid value of lower than 20 mgKOH/g or higher than 150 mgKOH/g, is used as the fixing agent. These absorbent bodies have a fixing ratio 2 of at most 65 in the fixation evaluation although the fixing ratio 1 thereof is 95 or more.

The absorbent bodies No. 13 and 14 are the cases where a synthetic rubber-based hot melt adhesive is used as the fixing agent. These synthetic rubber-based hot melt adhesives have low affinity to the water absorbent resin powder, and thus the fixing ratio is low. In particular, in the absorbent body No. 14, even if the amount of the synthetic rubber-based hot melt adhesive is increased, the fixing ratio does not improve so much.

**Industrial Applicability**

The absorbent body according to the present invention can be suitably used for, for example, absorbent articles used for absorbing body fluid excreted from human body, in particular suitably used for absorbent articles such as an incontinence pad, a disposable diaper and a sanitary napkin.

**Reference Signs List**

1: disposable diaper (absorbent article), 2: front abdominal portion, 3: back portion, 4: crotch portion, 5: notch, 6: exterior sheet material, 7: end edge, 8: front-side waist elastic member, 9: back-side waist elastic member, 10: front-side leg elastic member, 11: back-side leg elastic member, 12: front-side torso circumference elastic member, 13: back-side torso circumference elastic member, 15: front-side end holding sheet, 16: back-side end holding sheet, 17: side sheet, 18: elastic member, 20: absorbent main body, 21: absorbent body, 22: liquid permeable top sheet, 23: liquid impermeable back sheet, 24: water absorbent resin powder, 25: fixing agent, 26, 27: base material

**Claims**

1. An absorbent body comprising: a base material and a water absorbent resin powder fixed on the base material by a fixing agent,
   wherein the base material is a fiber base material having a mass per unit area in a range from 10 g/m$^2$ to 500 g/m$^2$,
   a material of a fiber constituting the fiber base material contains at least one member selected from the group consisting of polyethylene, polypropylene and polyethylene telephthalate,
   the fixing agent is a copolymer having an acid value in a range from 50 mgKOH/g to 130 mgKOH/g and including a structural unit (X) derived from at least one monomer selected from the group consisting of an olefin and a vinyl aromatic compound and a structural unit (Y) derived from a monomer having an acidic group,
   the copolymer is a graft copolymer including a main chain having the structural unit (X) and a side chain having the structural unit (Y), and
   the monomer having an acidic group is at least one monomer selected from the group consisting of acrylic acid, methacrylic acid, maleic acid, fumaric acid and maleic anhydride.

2. The absorbent body according to claim 1, wherein a fiber constituting the fiber base material has a fineness in a range from 1.0 dtex to 5.0 dtex.

3. The absorbent body according to claim 1, wherein a fiber constituting the fiber base material is a crimped fiber.

4. The absorbent body according to claim 3, wherein a fiber constituting the fiber base material is an eccentric core-sheath type spontaneously crimped fiber including a core composed of a first component and a sheath composed of a second component, or a parallel type spontaneously crimped fiber including a first component and a second component.

5. The absorbent body according to claim 1, wherein the fiber base material is an air-through nonwoven fabric or an air-laid nonwoven fabric.

6. The absorbent body according to claim 1, wherein the water absorbent resin powder has a mass average particle size in a range from 200 micrometers to 550 micrometers, and
   wherein an amount of a particle having a particle size of 150 micrometers or less in the water absorbent resin powder is 10 mass % or less and an amount of a particle having a particle size of larger than 850 micrometers in the water absorbent resin powder is 10 mass % or less.

7. The absorbent body according to claim 1, wherein the water absorbent resin powder has an absorption ratio under a load of 2 kPa in a range from 6 g/g to 35 g/g.

8. The absorbent body according to claim 1, wherein the water absorbent resin powder has a water absorption speed measured by a vortex method in a range from 5 seconds to 100 seconds.

9. The absorbent body according to claim 1, wherein the fixing agent is comprised in an amount ranging from 0.3 part by mass to 5 parts by mass with respect to 100 parts by mass of the water absorbent resin powder.

**10.** The absorbent body according to claim 1, wherein the water absorbent resin powder has a mass per unit area in a range from 20 g/m 2 to 500 g/m 2.

**11.** The absorbent body according to claim 1, prepared by applying a water absorbent resin particle coated with the fixing agent on the base material followed by carrying out a heating treatment.

**12.** The absorbent body according to claim 1, the absorbent resin powder is treated with an inorganic fine particle.

**13.** A water absorbent resin powder used in the absorbent body according to claim 11, wherein the water absorbent resin particle constituting the water absorbent resin powder is coated with the fixing agent.

**14.** An absorbent article comprising the absorbent body according to any one of claims 1 to 12.

**Patentansprüche**

**1.** Absorbierender Körper, umfassend: ein Basismaterial und ein Wasser-absorbierendes Harzpulver, fixiert auf dem Basismaterial durch ein Fixiermittel, wobei das Basismaterial ein Faserbasismaterial mit einer Masse pro Flächeneinheit in einem Bereich von 10 g/m$^2$ bis 500 g/m$^2$ ist,

ein Material einer Faser, welche das Faserbasismaterial konstituiert, mindestens ein Mitglied enthält, ausgewählt aus der Gruppe, bestehend aus Polyethylen, Polypropylen und Polyethylenterephthalat,
wobei das Fixiermittel ein Copolymer mit einem Säurewert in einem Bereich von 50 mgKOH/g bis 130 mgKOH/g ist und eine Struktureinheit (X), abgeleitet von mindestens einem Monomer, ausgewählt aus der Gruppe, bestehend aus einem Olefin und einer vinylaromatischen Verbindung, und eine Struktureinheit (Y), abgeleitet von einem Monomer mit einer Säuregruppe, einschließt,
wobei das Copolymer ein Pfropfcopolymer ist, welches eine Hauptkette mit der Struktureinheit (X) und eine Seitenkette mit der Struktureinheit (Y) einschließt, und
das Monomer mit einer Säuregruppe mindestens ein Monomer ist, ausgewählt aus der Gruppe, bestehend aus Acrylsäure, Methacrylsäure, Maleinsäure, Fumarsäure und Maleinsäureanhydrid.

**2.** Absorbierender Körper gemäß Anspruch 1, wobei eine Faser, welche das Faserbasismaterial konstituiert, eine Feinheit in einem Bereich von 1,0 dtex bis 5,0 dtex aufweist.

**3.** Absorbierender Körper gemäß Anspruch 1, wobei eine Faser, welche das Faserbasismaterial konstituiert, eine gekräuselte Faser ist.

**4.** Absorbierender Körper gemäß Anspruch 3, wobei eine Faser, welche das Faserbasismaterial konstituiert, eine spontan gekräuselte Faser vom exzentrischen Kern-Mantel-Typ, einschließend einen Kern, aufgebaut aus einer ersten Komponente, und eine Hülle, aufgebaut aus einer zweiten Komponente, oder eine spontan gekräuselte Faser vom Parallel-Typ, einschließend eine erste Komponente und eine zweite Komponente, ist.

**5.** Absorbierender Körper gemäß Anspruch 1, wobei das Faserbasismaterial ein Luftdurchgangsvlies oder ein luftgelegtes Vlies ist.

**6.** Absorbierender Körper gemäß Anspruch 1, wobei das Wasser-absorbierende Harzpulver eine massendurchschnittliche Teilchengröße in einem Bereich von 200 $\mu$m bis 550 $\mu$m aufweist, und wobei eine Menge eines Teilchens mit einer Teilchengröße von 150 $\mu$m oder weniger in dem Wasser-absorbierenden Harzpulver 10 Masse-% oder weniger ist und eine Menge eines Teilchens mit einer Teilchengröße von größer als 850 $\mu$m in dem Wasser-absorbierenden Harzpulver 10 Masse-% oder weniger beträgt.

**7.** Absorbierender Körper gemäß Anspruch 1, wobei das Wasser-absorbierende Harzpulver ein Absorptionsverhältnis unter einer Belastung von 2 kPa in einem Bereich von 6 g/g bis 35 g/g aufweist.

**8.** Absorbierender Körper gemäß Anspruch 1, wobei das Wasser-absorbierende Harzpulver eine Wasserabsorptionsgeschwindigkeit, gemessen durch ein Vortexverfahren, in einem Bereich von 5 Sekunden bis 100 Sekunden aufweist.

**9.** Absorbierender Körper gemäß Anspruch 1, wobei das Fixiermittel in einer Menge im Bereich von 0,3 Masseteilen bis 5 Masseteilen bezüglich 100 Masseteilen des Wasser-absorbierenden Harzpulvers umfasst ist.

10. Absorbierender Körper gemäß Anspruch 1, wobei das Wasser-absorbierende Harzpulver eine Masse pro Flächeneinheit in einem Bereich von 20 g/m$^2$ bis 500 g/m$^2$ aufweist.

11. Absorbierender Körper gemäß Anspruch 1, hergestellt durch Aufbringen eines Wasser-absorbierenden Harzteilchens, beschichtet mit dem Fixiermittel, auf dem Basismaterial gefolgt von dem Durchführen einer Wärmebehandlung.

12. Absorbierender Körper gemäß Anspruch 1, wobei das absorbierende Harzpulver mit einem anorganischen feinen Teilchen behandelt worden ist.

13. Wasser-absorbierendes Harzpulver, verwendet in dem absorbierenden Körper gemäß Anspruch 11, wobei das Wasser-absorbierende Harzteilchen, welches das Wasser-absorbierende Harzpulver konstituiert, mit dem Fixiermittel beschichtet ist.

14. Absorbierender Gegenstand, umfassend den absorbierenden Körper gemäß einem der Ansprüche 1 bis 12.

**Revendications**

1. Corps absorbant comprenant: une matière de base et une poudre de résine absorbant l'eau fixée sur la matière de base par un agent de fixation,
dans lequel la matière de base est une matière de base fibreuse présentant une masse par unité de surface allant de 10 g/m$^2$ à 500 g/m$^2$,
une matière fibreuse constituant la matière de base fibreuse contient au moins un élément sélectionné parmi le groupe consistant en polyéthylène, polypropylène et polyéthylène téréphtalate.
l'agent de fixation est un copolymère présentant une valeur d'acide allant de 50 mgKOH/g à 130 mgKOH/g et comprenant un motif structurel (X) dérivé d'au moins un monomère sélectionné parmi le groupe consistant en une oléfine et un composé vinylaromatique et un motif structurel (Y) dérivé d'un monomère ayant un groupe acide,
le copolymère est un copolymère greffé comprenant une chaîne principale présentant le motif structurel (X) et une chaîne latérale présentant le motif structurel (Y), et
le monomère ayant un groupe acide est au moins un monomère sélectionné parmi le groupe consistant en un acide acrylique, un acide méthacrylique, un acide maléique, un acide fumarique et un anhydride maléique.

2. Corps absorbant selon la revendication 1, dans lequel une fibre constituant la matière de base fibreuse présente une finesse allant de 1,0 dtex à 5,0 dtex.

3. Corps absorbant selon la revendication 1, dans lequel une fibre constituant la matière de base fibreuse est une fibre crêpée.

4. Corps absorbant selon la revendication 3, dans lequel une fibre constituant la matière de base fibreuse est une fibre spontanément crêpée de type cœur-gaine excentrique comprenant un cœur composé d'un premier composant et une gaine composée d'un deuxième composant, ou une fibre spontanément crêpée de type parallèle comprenant un premier composant et un deuxième composant.

5. Corps absorbant selon la revendication 1, dans lequel la matière de base fibreuse est un tissu non tissé à passage d'air ou un tissu non tissé air-laid.

6. Corps absorbant selon la revendication 1, dans lequel la poudre de résine absorbant l'eau présente une dimension particulaire moyenne en masse allant de 200 micromètres à 550 micromètres, et
dans lequel la quantité de particule présentant une dimension particulaire de 150 micromètres ou moins dans la poudre de résine absorbant l'eau, est de 10% en masse ou moins et la quantité de particule présentant une dimension particulaire supérieure à 850 micromètres dans la poudre de résine absorbant l'eau, est de 10% en masse ou moins.

7. Corps absorbant selon la revendication 1, dans lequel la poudre de résine absorbant l'eau présente un taux d'absorption sous charge de 2 kPa, allant de 6 g/g à 35 g/g.

8. Corps absorbant selon la revendication 1, dans lequel la poudre de résine absorbant l'eau présente une vitesse d'absorption de l'eau mesurée par méthode à vortex allant de 5 secondes à 100 secondes.

9. Corps absorbant selon la revendication 1, dans lequel l'agent de fixation est compris dans une quantité allant de 0,3 partie en masse à 5 parties en masse, sur base de 100 parties en masse de la poudre de résine absorbant l'eau.

10. Corps absorbant selon la revendication 1, dans lequel la poudre de résine absorbant l'eau présente une masse par unité de surface allant de 20 g/m$^2$ à 500 g/m$^2$.

11. Corps absorbant selon la revendication 1 préparé par application d'une particule de résine absorbant l'eau enrobée de l'agent de fixation sur la matière de base suivie de la réalisation d'un traitement thermique.

12. Corps absorbant selon la revendication 1, dans lequel la poudre de résine absorbante est traitée avec une particule fine inorganique.

13. Poudre de résine absorbant l'eau utilisée dans le corps absorbant selon la revendication 11, dans laquelle la particule de résine absorbant l'eau constituant la poudre de résine absorbant l'eau est enrobée de l'agent de fixation.

14. Article absorbant comprenant le corps absorbant selon l'une quelconque des revendications 1 à 12.

[Fig. 1]

[Fig. 2]

[Fig. 3]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 01089439 A
- JP 2015100610 A
- JP 2003225565 A
- JP 2006131767 A

**Non-patent literature cited in the description**

- Perry's Chemical Engineers Handbook. The Mc-Graw-Hill Companies, 1984, 21